# FASCICULE DE BREVET EUROPEEN

(11) **EP 0 485 265 B1**
(45) Date de publication et mention de la délivrance du brevet: **22.07.1998**
(21) Numéro de dépôt: 91402939.2
(22) Date de dépôt: 04.11.1991
(51) Int. Cl.: G06T 11/00

(54) **Dispositif et procédé de contrôle non destructif à acquisition simultanée de données radiographiques et de données tomographiques**
Verfahren und Methode zur zerstörungsfreien Prüfung mit gleichzeitiger Aufnahme von Röntgendaten und tomographischen Daten
Apparatus and method for non-destructive control with simultaneous acquisition of radiographic and tomographic data

(30) Priorité: 05.11.1990 FR 9013660
(43) Date de publication de la demande: 13.05.1992
(73) Titulaire: COMMISSARIAT A L'ENERGIE ATOMIQUE, 75015 Paris (FR)
(72) Inventeur: Peyret, Olivier, F-38000 Grenoble (FR); Thomas, Gérard, F-38800 Pont de Claix (FR)
(74) Mandataire: Lhoste, Catherine

(56) Documents cités:
- EP-A- 176 314
- EP-A- 292 402
- EP-A- 365 979
- GB-A- 2 169 180
- PATENT ABSTRACTS OF JAPAN vol. 10, no. 180 (P-471)(2236), 24 juin 1986; & JP - A - 61028850

## Description

L'invention se rapporte à un dispositif et à un procédé de contrôle non destructif à acquisition simultanée de données radiographiques et de données tomographiques. Ce dispositif peut être utilisé pour des applications industrielles ou médicales. Il utilise les techniques de la radiographie (rayons X ou gamma) et permet d'analyser la structure interne d'objets de dimensions et de masse variables et permet, en particulier, la détection et l'analyse d'éventuels défauts. Il peut fonctionner dans une large gamme d'énergie de rayonnement et en particulier dans une gamme allant de 1 KeV à 25 MeV, gamme recouvrant celle utilisée classiquement en tomographie ou radiographie médicale.

Parmi ces techniques, la présente invention s'intéresse plus particulièrement à celles permettant l'acquisition d'images radiographiques directement numérisées (radiographie numérique).

L'invention s'applique en particulier dans les domaines de l'automobile, de l'aéronautique, du spatial, du nucléaire.

Tout dispositif connu de radiographie numérique comprend une source de rayonnement X ou gamma et un détecteur de rayonnement, associé à une électronique d'acquisition et de conversion analogique-numérique de l'image détectée. Un système informatique assure ensuite la gestion (mémorisation, traitement, cumul, visualisation) des images numériques.

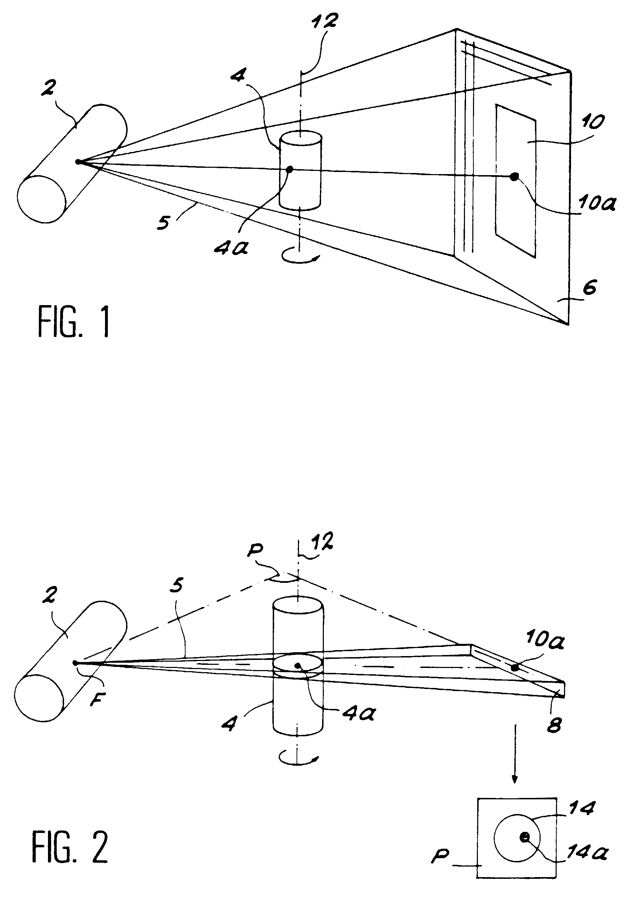

De façon connue, l'objet à radiographier étant placé entre la source et le détecteur, on peut obtenir, suivant le dispositif utilisé, au moins deux types d'images, une projection radiographique telle que représentée sur la figure 1 et une coupe tomographique telle que représentée sur la figure 2.

Sur ces figures 1 et 2, les références 2 et 4 représentent respectivement la source de rayonnement (X ou gamma) et l'objet à radiographier. La référence 4a indique un défaut dans l'objet et la référence 5 représente le rayonnement issu de la source. Dans le dispositif de la figure 2, ce rayonnement est collimaté. Les références 6 et 8 représentent respectivement un détecteur bidimensionnel à n lignes et p colonnes et donc une matrice de np points détecteurs, appelé détecteur 2D, et un détecteur linéaire à 1 ligne et p points, appelé détecteur 1D, n et p étant des entiers naturels supérieurs à 2 et généralement compris entre quelques dizaines et quelques milliers.

En projection radiographique (figure 1) l'ensemble source-objet-détecteur est fixe et on obtient une image radiographique 10 qui est une représentation bidimensionnelle de la transmission du rayonnement X ou gamma à travers l'objet 4 et constitue une projection conique de cet objet sur le plan du détecteur. La référence 10a représente l'image du défaut 4a de l'objet.

Cette image de transmission peut être convertie en une image représentant en chaque point, l'atténuation du rayonnement X ou gamma par l'objet.

Par une rotation de l'objet ou de l'ensemble source-détecteur autour de l'objet, selon un axe 12 transversal au rayonnement 5, on permet l'acquisition d'images de l'objet suivant différentes incidences qui peuvent être nécessaires à son contrôle. Un exemple de dispositif fonctionnant selon le principe de la figure 1 est décrit dans le document FR-A-2 574 583 déposé le 7 décembre 1984 au nom du demandeur.

En coupe tomographique (figure 2), il existe un mouvement de rotation relatif selon l'axe 12 entre l'ensemble source-détecteur et l'objet. On définit alors un plan perpendiculaire P à l'axe de rotation 12 passant par le foyer F de la source 2 et par le détecteur 8, appelé plan de coupe. Lors de la rotation, on peut acquérir dans ce plan différentes séries de mesures de l'atténuation du rayonnement par l'objet. Ces mesures sont mises en mémoire et utilisées pour reconstruire, grâce à un algorithme de calcul adapté, une vue tomographique 14 de l'objet qui constitue une coupe transversale de cet objet dans le plan P. La référence 14a représente une coupe du défaut 4a.

Cette coupe permet d'une part une étude morphologique précise de l'objet, d'autre part, des mesures de densité en chaque point de l'objet dans le cas où l'énergie du rayonnement est bien adaptée.

Il existe un certain nombre de dispositifs de tomographie dans le domaine médical et industriel. On peut, en particulier. mentionner celui du document FR-A-2 512 553 déposé le 10 septembre 1981 au nom du demandeur, appliqué au domaine industriel.

Le document GB-A-2 169 180 décrit un dispositif/procédé permettant l'extraction de données correspondant aux zones particulières d'un objet. Dans ce dispositif/procédé des coupes tomographiques sont réalisées pour une tranche donnée d'un objet à examiner contenant des éléments avec des coefficients d'absorption différents en utilisant un détecteur unidimensionnel. Deux acquisitions de données sont effectuées, une première en irradiation forte, une deuxième en irradiation faible. Pour chaque acquisition les données d'image sont stockées après une convolution et projection inverse. A partir de ces données pour chaque acquisition un sinogramme de différence est préparé pour l'image entière de cette acquisition. Les données du sinogramme de différence sont utilisées pour la reconstruction d'images.

De façon plus précise, l'invention s'intéresse aux dispositifs permettant l'acquisition en temps réel d'images radiographiques directement numérisées et utilisant un détecteur bidimensionnel (2D) de rayonnement X ou gamma.

Parmi ces détecteurs 2D, on peut citer de manière non limitative :
- les tubes intensificateur d'images radiographiques (ou amplificateurs de brillance) associés à une caméra vidéo ;
- les écrans radioluminescents optiquement stimulables dont la lecture est assurée par un faisceau laser, le signal issu des écrans étant reçu sur un photomultiplicateur (procédé FUJI) ;
- les écrans radioluminescents associés à une optique de reprise de l'image et à une caméra bas niveau de lumière.

Plusieurs documents publiés décrivent ce dernier type de détecteur et s'intéressent, en particulier, à l'amélioration des performances de l'écran radioluminescent. On peut notamment citer les références suivantes : FR-A-2 463 420 du 14 août 1979 décrivant l'utilisation d'un tel détecteur pour le contrôle de structures en béton armé et proposant une structure particulière de l'écran radioluminescent, FR-A-2 412 855 du 22 décembre 1978 et FR-A-2 574 583 du 7 décembre 1984 proposant différents procédés de fabrication de tels écrans et décrivant des dispositifs pour la mise en oeuvre de ces procédés.

Jusqu'à ce jour, pour le contrôle non destructif, on réalise couramment un certain nombre de projections radiographiques 10 d'un objet à contrôler suivant le procédé décrit en référence à la figure 1. Lorsqu'un défaut 4a est détecté dans une zone déterminée de l'objet, il peut y avoir alors un intérêt à réaliser une coupe tomographique de l'objet en vue d'une meilleure localisation de ce défaut et une mesure éventuelle de la densité du défaut.

On soumet alors l'objet à de nouvelles irradiations X ou gamma afin de réaliser des coupes tomographiques. Il s'ensuit une augmentation de la durée du contrôle non destructif, ce qui est contraire aux cadences industrielles et la nécessité d'utiliser deux types d'appareils distincts, ce qui pose des problèmes d'encombrement et complique la manutention des objets à contrôler. Ceci entraîne en outre un coût élevé du contrôle

Par ailleurs, lorsque l'objet à contrôler est un être humain, il est souhaitable de réduire le temps d'irradiation au maximum.

Pour diminuer la durée d'irradiation, il existe une solution complexe. Elle consiste, à partir de mesures bidimensionnelles de l'atténuation du rayonnement par un objet et des algorithmes spécifiques, de reconstruire l'ensemble du volume de l'objet et de réaliser ainsi un dispositif d'imagerie tridimensionnelle. En particulier, le document FR-A-2 615 619 du 21 mai 1987 déposé au nom du demandeur décrit un tel dispositif utilisant un algorithme spécifique.

A partir de l'image reconstruite du volume, il est donc évident qu'avec un tel système, on peut extraire n'importe quel plan représentant une coupe de l'objet, et disposer ainsi d'images de projections ou de coupes de l'objet.

Cependant, un tel système met en oeuvre une architecture électronique et informatique complexe qui s'adapte difficilement aux exigences de coût et de cadence du contrôle industriel. En effet, le nombre de données à traiter dans un temps qui doit rester raisonnable conduit d'une part à l'utilisation d'un calculateur de hautes performances et, d'autre part, à la réalisation d'opérateurs de reconstruction utilisant un grand nombre de processeurs (ou calculateurs parallèles) dont le côut est élevé.

Malgré cela, les temps de reconstruction restent aujourd'hui importants. Typiquement, pour un volume 128x128x128 avec 256 projections (128 étant le nombre de points selon une direction de l'espace), on obtient l'image du volume en 5 heures sur un VAX 6300, en 15 minutes sur un CRAY 2.

De plus, pour reconstruire l'ensemble du volume avec le moins possible d'artefacts, il peut être nécessaire de permettre des acquisitions avec un mouvement relatif complexe de l'ensemble source-détecteur par rapport à l'objet, conduisant à un ensemble mécanique coûteux.

L'invention a justement pour objet un dispositif et un procédé de contrôle non destructif à acquisition simultanée de données de projections radiographiques et de données de coupes tomographiques permettant de remédier aux inconvénients précédents.

Elle permet d'une manière simple, rapide et peu coûteuse l'acquisition de données radiographiques et de données tomographiques avec un même dispositif. En outre, elle permet une réduction de la durée d'exposition de l'objet à contrôler aux rayonnements ionisants. Elle est utilisable aussi bien dans les domaines industriels que médicaux.

La présente invention repose sur la possibilité d'extraire, en cours d'acquisition ou en différé, des données de projections radiographiques - en nombre limité, mais suffisant - de l'objet, de cumuler, éventuellement de compléter, et de structurer ces données afin de constituer le sinogramme d'une acquisition tomographique nécessaire à la reconstruction d'une coupe de cet objet.

Aussi, l'invention a pour objet un dispositif de contrôle non destructif à acquisition simultanée de données de projections radiographiques d'un objet et de données de coupes tomographiques de cet objet, comportant un ensemble source-détecteur de rayonnement ionisant, un support d'objet placé entre la source et le détecteur, des moyens pour assurer au moins un mouvement relatif de rotation du support par rapport à l'ensemble source-détecteur, le détecteur étant un détecteur bidimensionnel à plusieurs lignes et plusieurs colonnes, délivrant pour chaque point de chaque projection radiographique un signal analogique proportionnel à la quantité de rayonnement transmis par l'objet, un convertisseur analogique-numérique pour transformer les signaux fournis par le détecteur en des données numériques, une première mémoire pour stocker ces données numériques, un système d'extraction et de cumul d'une partie de ces données stockées, correspondant à au moins une zone particulière à contrôler de l'objet, une seconde mémoire pour le stockage des sinogrammes obtenus à partir des données sélectionnées et cumulées par le système d'extraction et un système de traitement de ces sinogrammes pour la reconstruction des coupes tomographiques.

L'invention a l'avantage de pouvoir être mise en oeuvre aisément avec tout système actuel d'acquisition en temps réel d'images numériques, utilisant un détecteur 2D de rayonnement X ou gamma.

Par rayonnement ionisant, il faut comprendre un rayonnement X ou gamma.

Un sinogramme est une matrice de j lignes et p colonnes (j.p points) où j correspond au nombre de projections radiographiques à des angles de rotation successifs de pas angulaire constant et p correspond au nombre de points d'une ligne de détecteurs.

Dans le cas d'un détecteur linéaire utilisé en tomographie classique, chaque ligne du sinogramme correspond à l'information, pour une projection donnée, de la ligne du détecteur.

Dans le cas de l'invention utilisant un détecteur 2D, chaque ligne du sinogramme correspond à l'information, pour une projection donnée, d'une ou de plusieurs lignes cumulées (somme des points de même abscisse desdites lignes) du détecteur.

Le dispositif de l'invention est un dispositif polyvalent permettant de réaliser à la fois des projections radiographiques et des coupes tomographiques d'un même objet ; il permet de réaliser, à partir d'une même séquence d'acquisition, des vues en projection et des coupes d'un même objet.

L'invention permet en outre de réaliser des coupes tomographiques en choisissant, après acquisition des données, le plan de coupe et la hauteur de coupe. Ainsi, à partir d'une même séquence d'acquisition, on peut réaliser plusieurs coupes tomographiques de l'objet dans des plans de coupe différents ou dans un même plan de coupe avec des hauteurs de coupe différentes, en particulier pour réaliser le meilleur compromis résolution spatiale/rapport signal sur bruit adapté à la visualisation d'un détail ; la hauteur de coupe correspond au nombre de lignes sélectionné et le plan de coupe est défini par le rang de la ligne centrale sélectionnée.

Enfin, elle limite la durée d'irradiation de l'objet puisqu'elle utilise les mêmes données pour les deux types d'image.

Avec le système de l'invention, différents processus de contrôle non destructif d'un objet peuvent être mis en oeuvre. En particulier, il est possible de traiter les informations fournies par le détecteur en temps différé ou en temps réel. De plus, ce dispositif original peut être utilisé en mode radiographique classique (acquisition d'informations, mémorisation d'une ou plusieurs vues en projection par le circuit de traitement) ou en mode tomographique classique (collimation du rayonnement, acquisition d'informations et constitution d'un sinogramme à partir d'un grand nombre de projections).

Dans ce dernier cas, la hauteur de coupe est avantageusement réalisée à l'aide d'une collimation primaire du faisceau de rayonnement émis par la source, limitant le rayonnement diffusé de l'objet.

De plus, l'utilisation d'un détecteur 2D dans le cas de la tomographie classique donne la possibilité de correction du rayonnement diffusé par l'objet. On utilise alors les lignes adjacentes aux lignes définissant la coupe. Ces lignes adjacentes ne recouvrent en effet que le rayonnement diffusé par l'objet.

Pour de plus amples détails sur la correction du rayonnement diffusé, on peut se référer au document FR-A-2 504 278 déposé le 15 avril 1981 au nom du demandeur.

Selon un mode de réalisation du dispositif de l'invention, le système de traitement comporte un calculateur pour permettre la gestion de l'ensemble du dispositif et la reconstruction des coupes tomographiques. Ce calculateur assure en particulier la gestion des différentes mémoires du dispositif ainsi que du système d'extraction. Il peut également traiter les données issues du détecteur pour effectuer des corrections.

De façon avantageuse, ce calculateur commande un appareil de visualisation des projections radiographiques et/ou des coupes tomographiques et un système d'accès à ce calculateur. Ce calculateur assure en outre la commande en rotation de l'objet ou de l'ensemble source-détecteur.

Ce calculateur peut être complètement intégré au dispositif de l'invention ou être constitué par un premier calculateur, intégré au dispositif pour en assurer sa gestion, et par un deuxième calculateur, externe au dispositif (situé par exemple dans un dispositif annexe tel qu'un tomographe), permettant la reconstruction des coupes tomographiques.

La première mémoire du dispositif peut permettre de stocker et de cumuler les trames successives d'une même projection afin d'améliorer la qualité de chaque projection radiographique. En particulier, lorsque le détecteur travaille au rythme vidéo.

L'appareil de visualisation peut permettre de visualiser chaque projection radiographique au fur et à mesure de leur acquisition.

De préférence, un second dispositif de collimation est prévu en regard du détecteur pour servir de masque au rayonnement issu directement de la source. Ceci est notamment le cas, lorsque l'objet à contrôler est plus petit que le détecteur.

Avantageusement, des moyens d'archivage des projections radiographiques et/ou des coupes tomographiques de l'objet sont prévus. Ces moyens peuvent consister en des bandes magnétiques, des disques ou des disquettes. Ces moyens sont reliés au calculateur.

L'invention a aussi pour objet un procédé de contrôle non destructif d'un objet à acquisition simultanée de données e projections radiographiques et de données de coupes tomographiques de cet objet, utilisant le dispositif décrit précédemment.

Ce procédé consiste à :
a) - soumettre ledit objet à un rayonnement ionisant sous différentes incidences, l'objet ayant au moins un mouvement relatif de rotation par rapport à l'ensemble source-détecteur, chaque incidence correspondant à une projection,
b) - détecter le rayonnement transmis par l'objet à l'aide d'un détecteur bidimensionnel à plusieurs lignes et colonnes, , délivrant en chaque point des signaux analogiques proportionnels à la quantité de rayonnement transmis par l'objet,
c) - convertir lesdits signaux analogiques en données numériques,
d) - mémoriser ces données numériques dans une première mémoire,
e) - traiter ces données numériques pour établir des projections radiographiques de l'objet,
f) - sélectionner au moins une zone particulière à contrôler de l'objet,
g) - extraire de la première mémoire les données numériques correspondant à ladite zone et les cumuler, pour chaque projection,
h) - réaliser le sinogramme correspondant à ladite zone à partir des données extraites et cumulées des différentes projections,
i) - traiter ce sinogramme pour reconstruire la coupe tomographique de l'objet dans la zone sélectionnée.

Une zone sélectionnée est définie par le plan de coupe et la hauteur de coupe.

Le procédé de l'invention permet d'obtenir autant de sinogrammes et donc de coupes tomographiques que de zones sélectionnées.

L'ordre exact des étapes du procédé est fonction notamment du mode de fonctionnement choisi pour le dispositif (par exemple fonctionnement en temps différé ou temps réel).

En particulier, l'étape g consiste à extraire de la première mémoire n' lignes des j projections radiographiques, de part et d'autre de la ligne de ces projections correspondant au milieu de ladite zone particulière et de cumuler les 2n'+1 lignes, colonne à colonne, pour obtenir j lignes correspondant aux j projections et constituant chacune une ligne du sinogramme.

D'autres caractéristiques et avantages de l'invention ressortiront mieux de la description qui va suivre, donnée à titre purement illustratif et non limitatif.

La description se réfère aux dessins annexés dans lesquels :
- la figure 1, déjà décrite, représente schématiquement le principe de fonctionnement d'un dispositif de contrôle à projections radiographiques, conformément à l'art antérieur,
- la figure 2, déjà décrite, représente schématiquement le principe de fonctionnement d'un dispositif de contrôle à coupes tomographiques, conformément à l'art antérieur,
- la figure 3 est un synoptique général du dispositif de contrôle conforme à l'invention,
- la figure 4 illustre le principe de traitement des informations par le dispositif conforme à l'invention,
- la figure 5 illustre un mode de réalisation du dispositif de l'invention pour le domaine médical, et
- les figures 6A et 6B sont des synoptiques des différentes étapes de fonctionnement du dispositif de l'invention, respectivement en mode temps différé et en mode temps réel.

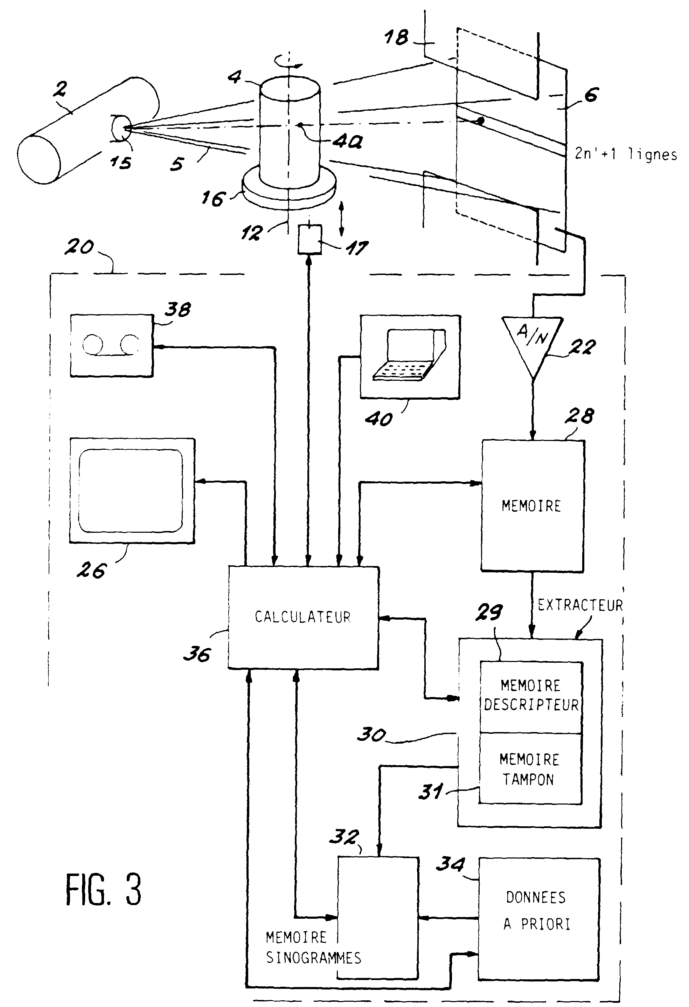

En référence aux figures 3 à 5, le dispositif conforme à l'invention comprend une source 2 de rayonnement X ou gamma suivant le domaine d'énergie privilégiée, telle qu'un tube de rayonnement X, une source radioactive, un accélérateur linéaire. Suivant le dispositif, l'énergie du rayonnement X ou gamma peut être ajustable. Un collimateur primaire 15 (facultatif) peut être placé en sortie de la source dans le cas de l'utilisation du mode tomographique classique.

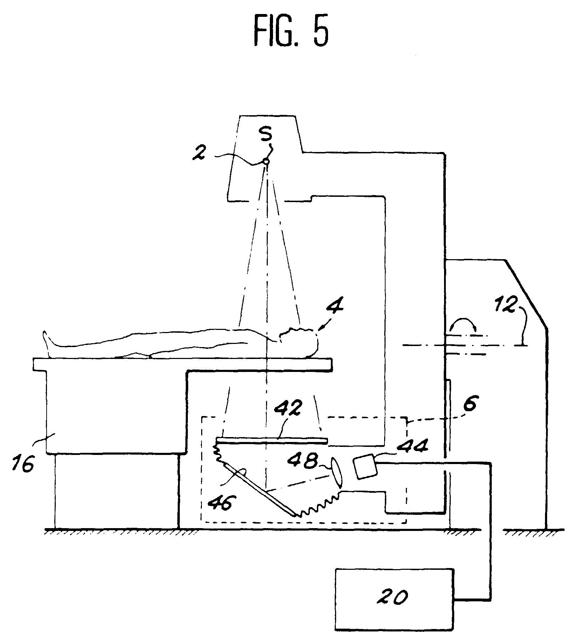

L'objet à radiographier 4 est lié à un support mobile 16 commandé au moins en rotation selon l'axe 12 par un système mécanique 17. Dans une application de type médical (figure 5), on préfère que le mouvement de rotation soit réalisé par l'ensemble source-détecteur, le patient restant fixe.

Le système de déplacement 17 peut permettre aussi par exemple une translation selon l'axe de la rotation.

A chaque orientation et position de l'objet par rapport à l'ensemble source-détecteur, correspond une seule projection radiographique.

L'ensemble de détection bidimensionnel 6 du rayonnement mesure la quantité de rayonnement (X ou gamma) en chaque point, et fournit une matrice de données analogiques.

Cet ensemble de détection comporte n lignes et p colonnes définissant np points-image (typiquement 512² ou 1024² points). Il permet l'acquisition simultanément des données de projections radiographiques et des données de coupes tomographiques. Un exemple de détecteur sera décrit ultérieurement.

Un collimateur secondaire 18 (facultatif), en face avant du détecteur 6, peut être utilisé pour servir de masque au rayonnement direct provenant de la source 2, évitant ainsi la saturation des points-image concernés.

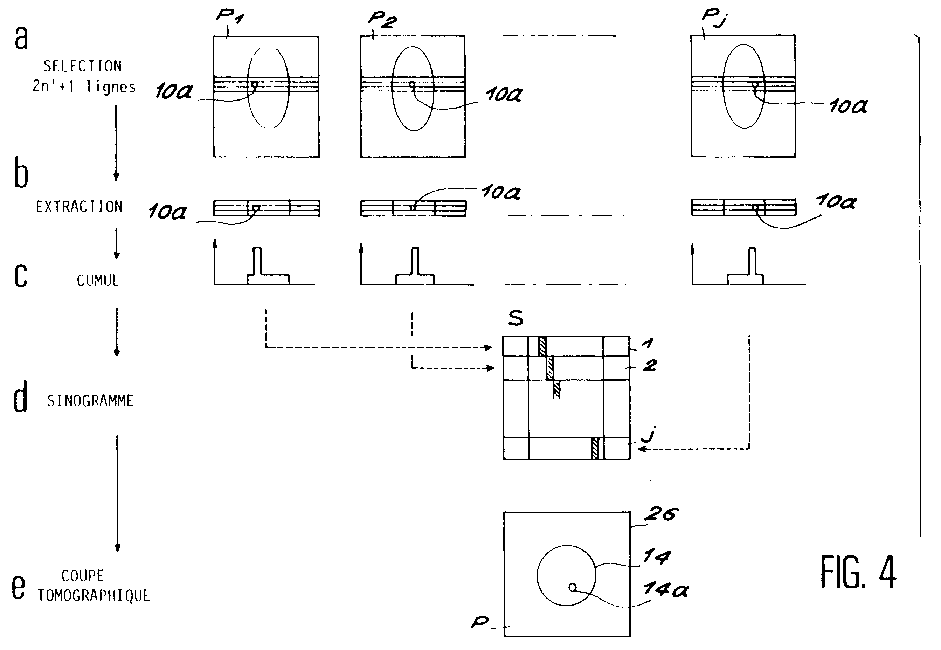

L'ensemble mécano-optique ci-dessus est associé à un système 20 de traitement d'images, connecté en sortie du détecteur, qui permet l'établissement simultané de projections radiographiques et de coupes tomographiques (figure 4).

Cet ensemble comprend un convertisseur 22 analogique-numérique transformant les signaux analogiques fournis par le détecteur en données numériques.

Une mémoire de stockage d'images 28 permet d'enregistrer lors d'une séquence d'acquisitions une projection radiographique en mode temps réel à la fois, ou différentes projections radiographiques P1, P2, ..., Pj en mode temps différé (partie a de la figure 4) avec j entier, allant typiquement de 1 à quelques centaines. Sa capacité est calculée en fonction du nombre maximum de projections et de la taille mémoire d'une image (nombre de points, codages).

Cette mémoire 28 peut permettre de cumuler un certain nombre de trames d'informations, aussi bien en mode temps réel qu'en mode temps différé lors de l'acquisition d'une projection radiographique dans une direction donnée (ce nombre d'images élémentaires étant défini par l'opérateur) afin d'augmenter la qualité des projections.

Un système de visualisation 26 (moniteur du calculateur 36 par exemple) permet la visualisation des trames d'informations en cours d'acquisition (mode radioscopique) et des trames d'informations cumulées, qui constituent les projections radiographiques (mode radiographique). Il permet en outre la visualisation des coupes tomographiques en mode temps réel ou différé.

La visualisation simultanée des projections radiographiques et de coupes tomographiques peut être réalisée sur un même écran ou sur deux écrans.

Un système 30 d'extraction et de cumul (opérateur spécialisé ou logiciel) permet d'extraire rapidement (étape b, figure 4) des différents plans-image P1, ..., Pj, les 2n'+1 lignes d'informations sélectionnées par l'opérateur pour chaque zone d'intérêt (avec 2n'+1 entier≤n) dans la mémoire de stockage d'images 28 soit au fur et à mesure de l'acquisition des projections, la ou les zones d'intérêt étant définies par l'opérateur ou par le système avant l'acquisition, soit après l'acquisition la ou les zones d'intérêt étant alors définies par l'opérateur après l'acquisition.

Le système d'extraction 30 réalise ensuite un cumul (étape c, figure 4) des 2n'+1 lignes d'informations sélectionnées dans chaque projection radiographique P1, P2, ..., Pj et pour chaque zone d'intérêt (addition des points de même abscisse dans chacune des j projections).

Le graphe c de la figure 4 est en fait une projection du sinogramme de la partie d de la figure 4.

Le système d'extraction constitue en fait tout ou partie du sinogramme S en chargeant la mémoire de sinogrammes 32.

Dans cette mémoire peuvent être stockées des données a priori sur l'objet radiographié (stockées au préalable dans une mémoire 34) pour enrichir le sinogramme. Un calculateur 36 utilise les données de la mémoire 32 pour reconstruire à l'aide d'un algorithme adapté la ou les coupes tomographiques désirées (étape e, figure 4) que l'on visualise sur l'écran d'affichage 26.

L'ordinateur 36 assure en outre le pilotage des moyens 17 de déplacement du support d'objet et la gestion des moyens 28, 29.

Dans le cas où ce calculateur 36 ne permet pas de reconstruire les coupes tomographiques, la mémoire 32 est constituée par un support mobile (disque, disquette, bande magnétique) permettant le transfert des données de la mémoire 32 vers un calculateur de tomographe situé dans un autre lieu et équipé de son propre système de visualisation.

Des moyens d'archivage 38, du type bande magnétique, peuvent être associés au dispositif ainsi que des moyens de commande 40 tels qu'un terminal d'ordinateur.

Le détecteur selon l'invention peut par exemple être constitué, comme représenté sur la figure 5, d'un écran radioluminescent 42 au Gd₂0₂S dopé au terbium ou à l'europium, associé à une caméra vidéo . Dans l'application médicale représentée, l'écran 42 est parallèle au support 16 qui est ici transparent au rayonnement utilisé et parallèle au sol. Un miroir plan 46 doit être prévu pour réfléchir la lumière fournie par l'écran 42 vers l'entrée de la caméra après focalisation à l'aide d'un objectif 48. Le miroir 46 est placé à 45° par rapport à la normale à l'écran 42.

La sortie de la caméra est connectée directement au convertisseur analogique-numérique 22.

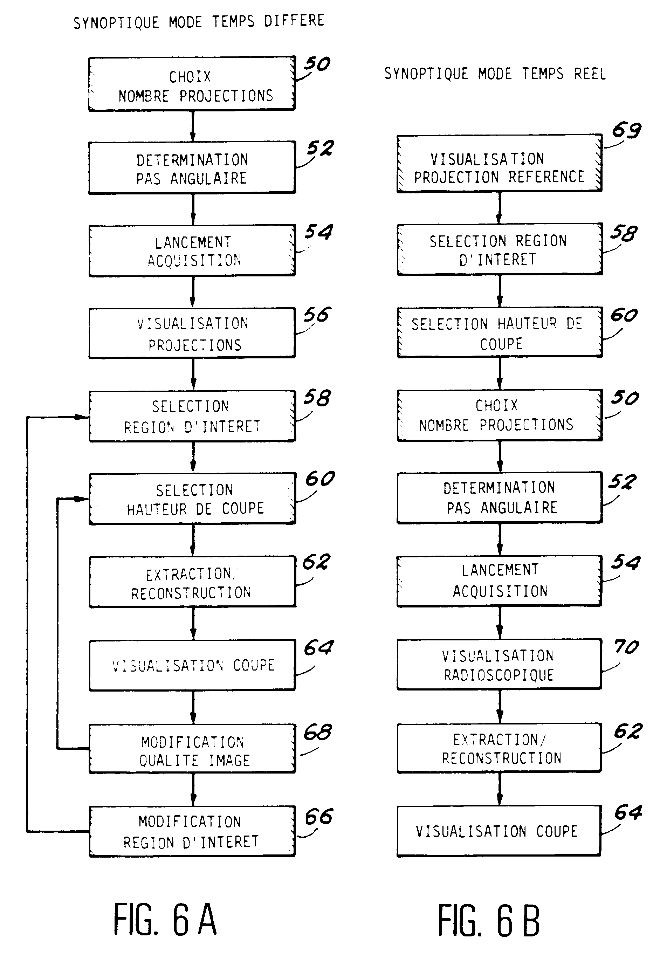

On décrit, ci-après, le fonctionnement du dispositif de l'invention en mode temps différé, figure 6A, puis en mode temps réel, figure 6B, dans le cas d'une application industrielle où c'est l'objet qui est mis en rotation. L'intervention d'un opérateur est indiquée par des hachures.

### Mode temps différé : il s'applique préférentiellement dans une logique d'expertise.

L'utilisateur détermine tout d'abord (étape 50) le nombre j de projections radiographiques à effectuer, le calculateur déduit automatiquement de j (étape 52) le pas angulaire de rotation de l'objet 4 autour de l'axe 12.

L'objet à contrôler est alors mis en rotation et l'utilisateur lance l'acquisition de plusieurs vues en projection de l'objet. La mise en rotation de l'objet coïncide avec le lancement de l'acquisition (étape 54). L'opérateur effectue alors une visualisation des projections au cours de l'acquisition (visualisation radioscopique) ou après l'acquisition (visualisation radiographique) (étape 56) sur l'afficheur 26, pour détecter un éventuel défaut 4a.

Un nombre minimum (j) de projections ayant été réalisé et ces projections ayant été mises en mémoire dans la mémoire 28, l'utilisateur veut réaliser une coupe tomographique dans la zone particulière où le défaut a été détecté. L'utilisateur définit alors (étape 58) une région autour du défaut détecté (rang de la ligne centrale du défaut par exemple) puis définit (étape 60) une hauteur de coupe (2n'+1 lignes, soit n' lignes sélectionnées de part et d'autre de cette ligne centrale de référence), en fonction des performances désirées (résolution spatiale, rapport signal sur bruit).

Si les conditions géométriques (par exemple source éloignée du détecteur avec faible grandissement ou petit corps à étudier,...) du dispositif sont telles que la divergence du faisceau de rayonnement est faible (approximation projection parallèle), la région définie peut être éloignée du plan médian (perpendiculaire à l'axe de rotation 12 et passant par la source).

Les 2n'+1 lignes d'informations choisies sont extraites de chaque projection et cumulées point à point pour chaque projection (étape 62) à l'aide du système 30 d'extraction et de cumul. Chaque ligne d'informations cumulée est stockée dans la mémoire 32 pour constituer le sinogramme S d'une acquisition tomographique.

Le calculateur 36 à l'aide d'un algorithme de reconstruction classique réalise alors la coupe de l'objet (étape 64) dans le plan P défini (ou plan de coupe). Un algorithme de reconstruction en tomographie utilisable dans l'invention est par exemple celui décrit dans le livre intitulé : "Actualité en Radiodiagnostic" ; sous-titre "Nouvelles Technologies"; auteurs M. AMIEL et autres ; éditeur MASSON - année 1982 ; Chapître 3 (Nouvelles tomographies), pages 26 à 28.

L'utilisateur peut définir, pour une même série de projections, plusieurs régions d'intérêt et réaliser ainsi plusieurs coupes tomographiques dans des plans différents. Il peut donc modifier la région d'intérêt (étape 66) (modification du plan de coupe) ainsi que la qualité de chaque image (étape 68) (modification hauteur de coupe).

### Mode temps réel : il s'applique préférentiellement dans une logique de contrôle automatique.

L'objet à contrôler possède une ou des zones spécifiques pour lesquelles il est a priori intéressant de réaliser une coupe tomographique (zone d'intérêt particulier, zone de géométrie complexe, localisation d'un détail, etc., ...). Avant le contrôle, l'utilisateur visualise (étape 69) des projections radiographiques de référence (radiographies antérieures stockées sur la bande 38 par exemple) sur le moniteur 26 et sélectionne (étape 58) la ou les zones d'intérêt.

L'utilisateur peut alors définir une zone a priori dans l'image et une hauteur de coupe (étape 60) de la même manière que précédemment.

Après fixation de la hauteur de coupe, l'utilisateur fixe le nombre de projections (étape 50) et ensuite le calculateur en déduit le pas angulaire de la rotation de l'objet (étape 52), puis l'utilisateur lance l'acquisition (étape 54).

On peut faire (étape 70) sur le moniteur du contrôle en visualisation radioscopique de l'objet en rotation, mais dans ce mode les projections entières peuvent ne pas être conservées au-delà de cette visualisation. Seules les parties intéressantes de ces projections sont avantageusement mémorisées.

Seules les lignes d'informations concernées par la zone d'intérêt définie sont extraites et cumulées à l'aide du système 30 puis mises en mémoire (étape 62) dans la mémoire 32 pour constituer en temps réel le sinogramme de l'acquisition tomographique. A la fin de l'acquisition, les données sont immédiatement disponibles pour la reconstruction de la coupe tomographique (étape 64) grâce au calculateur 36 et pour être visualisées à leur tour sur le moniteur 26.

Dans ces deux modes, on peut, dans le cas où le nombre j de projections est faible, utiliser des techniques, par ailleurs connues, pour compléter les données lors de la constitution du sinogramme, en vue d'améliorer la qualité des coupes tomographiques. A titre d'exemple, on peut citer "Utilization of the classical reconstruction algorithm for incomplete data in X-ray" E. Tournier, Ph. Rizo, G. Thomas, ASNT Conference, 25-27 juillet, Seattle (1989).

On donne ci-après deux exemples d'utilisation de l'invention.

### 1. Dans une logique d'expertise (mode temps différé)

- Acquisition de projections radiographiques suivant différentes incidences en mode temps différé, de l'objet à expertiser,
- Visualisation de ces projections,
- Repérage d'un défaut ou d'un détail à contrôler,
   Sélections plan de coupe/hauteur de coupe,
   Réalisation d'une ou plusieurs coupes tomographiques à partir des données,
   Visualisation et analyse des coupes,
- (Facultatif)
   Le défaut justifie une analyse plus précise : passage en mode tomographique classique, collimation/ sélections plan de coupe/hauteur de coupe, nouvelle acquisition d'un grand nombre de projections, réalisation, visualisation et analyse d'une coupe tomographique optimisée.

### 2. Dans une logique de contrôle automatique (mode temps réel)

- Le contrôle automatique de pièces peut justifier la réalisation d'une ou plusieurs projections et d'une ou plusieurs coupes tomographiques dans une région sensible de l'objet, région préalablement connue,
- Sélection de cette région - plan de coupe/hauteur de coupe à partir de projections de référence (une pièce type),
- Pour chaque pièce, acquisition de projections radiographiques suivant différentes incidences en mode temps réel,
- Réalisation automatique de coupes tomographiques demandées,
- Analyse des projections radiographiques et de coupes tomographiques par le système,
- Suivant le résultat de l'analyse, acceptation ou rejet de la pièce.

### Choix du plan de coupe

Le plan de coupe est défini a posteriori dans le cas d'utilisation du mode temps différé. D'autre part, l'utilisation d'un détecteur 2D donne la possibilité d'au moins 2 axes de rotation de l'objet, suivant les conditions géométriques du dispositif. En effet, on peut sélectionner indifféremment des lignes et des colonnes d'informations dans l'image numérique, et le procédé décrit à partir d'une sélection de lignes peut être décrit de la même manière à partir d'une sélection de colonnes.

D'autre part, dans le cas où la divergence du faisceau de rayonnement (X ou gamma) n'est pas négligeable et que la zone sélectionnée est éloignée du plan médian (passant par le foyer de la source de rayonnement), on peut avoir au minimum une bonne estimation de la position d'un éventuel défaut dans le volume, en utilisant une reconstruction faisant appel aux lignes (de rang différent suivant la projection) où apparaît ce défaut.

### Choix de la hauteur de coupe

La hauteur de coupe correspond au nombre de lignes cumulées. Dans le mode d'analyse en temps différé, elle est définie a posteriori en fonction de la qualité de l'image désirée et en particulier, en fonction du compromis résolution spatiale/résolution en densité.

Un faible nombre de lignes (le nombre de lignes dépend de la taille du défaut recherché) permet une haute définition au détriment du rapport signal/bruit. Le cumul de plusieurs lignes permet un meilleur rapport signal/bruit au détriment de la résolution spatiale.

### Choix de l'énergie du rayonnement

Une attention particulière doit être portée concernant le choix de l'énergie du rayonnement X ou gamma utilisée. En effet, en fontion de l'objet à radiographier et des mesures à atteindre, l'énergie optimale peut ne pas être identique dans les différents modes de fonctionnement. Aussi, on peut utiliser un dispositif simple comportant une source monoénergétique qui réalise un compromis entre les différents modes ou, au contraire, utiliser un dispositif comportant une source polyénergétique avec un choix adapté de l'énergie en fonction du mode.

La description suivante fournit un exemple possible de réalisation du système d'extraction et de cumul 30 (ou extracteur).

L'extracteur est constitué de deux mémoires au minimum :
- Une mémoire-descripteur 29 dans laquelle sont enregistrés les paramètres de l'extraction fournis par l'opérateur :
   mode de l'extraction (temps réel ou temps différé),
   nombre de projections,
   nombre de zones d'intérêt sélectionnées,
   rang des lignes centrales d'informations localisant les zones,
   nombre de lignes sélectionnées par zone,
- Une mémoire-tampon 31 dans laquelle sont cumulées les lignes sélectionnées. Elle est constituée au minimum d'une ligne sur laquelle se cumulent les 2n'+1 lignes de chaque zone d'intérêt, pour chaque projection. La capacité de cette mémoire-tampon définit la vitesse de l'extraction (possibilité de réaliser en parallèle le cumul des lignes de plusieurs zones d'intérêt et en mode différé de plusieurs projections enregistrées dans la mémoire de stockage d'images).
- La mémoire de sinogrammes (indépendante ou non des mémoires précédentes) reçoit les lignes cumulées et constitue les sinogrammes par un adressage adéquat des lignes. Les sinagrammes peuvent être complétés par des données par exemple sur la forme, la densité de l'objet ... présentes a priori dans la mémoire de sinogrammes.

En mode temps réel, l'extracteur est synchronisé sur l'acquisition (ou irradiation) par le calculateur 36, les données sont récupérées directement dans la mémoire 28 au rythme de l'acquisition. La mémoire-tampon est structurée éventuellement pour cumuler en parallèle les lignes correspondant à différentes zones d'intérêt. Le transfert vers la mémoire de sinogrammes 32 se fait au rythme de l'acquisition.

A la fin de l'acquisition des projections, la mémoire de sinogrammes est chargée.

En mode temps différé, l'extracteur 30 travaille sur un rythme propre qui dépend de la capacité de la mémoire tampon, les données sont récupérées dans la mémoire de stockage d'images 28.

On peut citer au moins une application industrielle de l'invention, liée au contrôle non destructif pour des applications aérospatiales. Dans cette application :
- la source de rayonnement X est un accélérateur linéaire d'énergie de plusieurs MeV.
- l'objet est cylindrique et contient un carburant. On cherche à détecter des délaminages près de l'enveloppe et des bulles dans le volume.
- l'objet est animé d'un mouvement de rotation et de translation suivant l'axe de rotation 12 pour contrôler l'ensemble.
- le détecteur de rayonnement X est constitué, comme sur la figure 5, d'un écran radioluminescent 42 associé à un système optique grand champ (miroir 46) de reprise de l'image et à une caméra 44 bas niveau de lumière.

Le mode temps différé est bien adapté à ce contrôle pour permettre une bonne localisation et une mesure de la densité des défauts détectés lors de l'examen radioscopique.

## Revendications

1. Dispositif de contrôle non destructif à acquisition simultanée de données de projections radiographiques d'un objet et de données de coupes tomographiques de cet objet, comportant un ensemble source-détecteur (2-6) de rayonnement ionisant, un support (16) d'objet placé entre la source et le détecteur, des moyens (17) pour assurer au moins un mouvement relatif de rotation du support par rapport à l'ensemble source-détecteur, le détecteur étant un détecteur bidimensionnel à plusieurs lignes et plusieurs colonnes, délivrant pour chaque point de chaque projection radiographique (P1, ..., Pj) un signal analogique proportionnel à la quantité de rayonnement transmis par l'objet (4), un convertisseur (22) analogique-numérique pour transformer les signaux fournis par le détecteur en des données numériques, une première mémoire (28) pour stocker ces données numériques, un système (30) d'extraction et de cumul d'une partie de ces données stockées, correspondant à au moins une zone particulière à contrôler de l'objet, une seconde mémoire (32) pour le stockage des sinogrammes obtenus à partir des données sélectionnées et cumulées par le système d'extraction et un système de traitement (36) de ces sinogrammes pour la reconstruction des coupes tomographiques (14).

2. Dispositif selon la revendication 1, caractérisé en ce que le système de traitement comporte un calculateur (36) pour la gestion du dispositif et la reconstruction des coupes tomographiques, commandant un appareil de visualisation (26) des projections radiographiques (P1, ..., Pj) et/ou des coupes tomographiques (14) et un système d'accès (40) à ce calculateur.

3. Dispositif selon la revendication 1 ou 2, caractérisé en ce qu'un premier collimateur (15) est prévu en sortie de la source pour collimater le rayonnement (5) issu de la source pour réaliser de la tomographie classique.

4. Dispositif selon l'une quelconque des revendications 1 à 3, caractérisé en ce qu'un second collimateur (18) est prévu en regard du détecteur pour servir de masque au rayonnement issu directement de la source.

5. Dispositif selon l'une quelconque des revendications 1 à 4, caractérisé en ce que des moyens d'archivage (38) des projections radiographiques et/ou des coupes tomographiques de l'objet sont prévus.

6. Dispositif selon l'une quelconque des revendications 1 à 5, caractérisé en ce que le système d'extraction (30) comporte au moins deux mémoires, une mémoire (29) dans laquelle sont enregistrés des paramètres d'extraction de ladite partie des données et une mémoire (31) dans laquelle sont cumulées les lignes ou colonnes sélectionnées.

7. Procédé de contrôle non destructif d'un objet, consistant à :
a) - soumettre un objet (4) à un rayonnement ionisant (5) sous différentes incidences, l'objet ayant au moins un mouvement relatif de rotation par rapport à l'ensemble source-détecteur, chaque incidence correspondant à une projection,
b) - détecter le rayonnement transmis par l'objet à l'aide d'un détecteur bidimensionnel (6) à plusieurs lignes et colonnes, délivrant en chaque point des signaux analogiques proportionnels à la quantité de rayonnement transmis par l'objet,
c) - convertir (22) lesdits signaux analogiques en données numériques,
d) - mémoriser ces données numériques dans une première mémoire (24),
e) - traiter (36) ces données numériques pour établir des projections radiographiques (P1, ..., Pj) de l'objet,
f) - sélectionner au moins une zone particulière à contrôler de l'objet,
g) - extraire (30) de la première mémoire les données numériques correspondant à ladite zone et les cumuler, pour chaque projection,
h) - réaliser (32) le sinogramme correspondant à ladite zone à partir des données extraites et cumulées, des différentes projections,
i) - traiter (36) ce sinogramme pour reconstruire la coupe tomographique de l'objet (14) dans la zone sélectionnée.

8. Procédé selon la revendication 7, caractérisé en ce que l'on extrait de la première mémoire n' lignes ou colonnes de chaque projection radiographique de part et d'autre de la ligne ou colonne de ces projections correspondant au milieu de ladite zone particulière et qu'on les cumule colonne à colonne ou ligne à ligne dans chaque projection.

9. Procédé selon la revendication 7 ou 8, caractérisé en ce que l'on visualise sur un appareil de visualisation (26) les projections radiographiques (P1, ..., Pj) et/ou les coupes tomographiques (14).

## Patentansprüche

1. Vorrichtung zur zerstörungsfreien Prüfung mit gleichzeitiger Erfassung von Daten von Röntgenprojektionen eines Objekts und Daten von tomographischen Schnitten dieses Objekts, mit einer Quelle-Detektor-Anordnung (2-6) für ionisierende Strahlung, einem Objektträger (16), angeordnet zwischen der Quelle und dem Detektor, Einrichtungen (17), die wenigstens eine relative Drehbewegung des Trägers in bezug auf die Quelle-Detektor-Anordnung aufrechterhalten, wobei der Detektor ein zweidimensionaler Detektor mit mehreren Zeilen und mehreren Spalten ist, der für jeden Röntgenprojektionspunkt (P1, ..., Pj) ein zu der von dem Objekt (4) durchgelassenen Strahlung proportionales analoges Signal liefert, einem Analog-Digital-Wandler (22), um die durch den Detektor gelieferten Signale in digitale Daten umzuwandeln, einem ersten Speicher (28) zum Speichern dieser digitalen Daten, einem System (30) zum Abfragen bzw. Auslesen und Summieren eines Teils dieser gespeicherten Daten, wenigstens einer zu prüfenden speziellen Zone des Objekts entsprechend, einem zweiten Speicher (32) zum Abspeichern der Sinogramme aufgrund der durch das Ausles- bzw. Abfragsystem ausgewählten und summierten bzw. kumulierten Daten, und einem Verarbeitungssystem (36) dieser Sinogramme zur Rekonstruktion der tomographischen Schnitte (14).

2. Verfahren nach Anspruch 1, dadurch gekennzeichnet, daß das Verarbeitungssystem einen Rechner (36) zur Verwaltung der Vorrichtung und der Rekonstruktion der tomographischen Schnitte umfaßt, der ein Gerät (26) zur Sichtbarmachung der Röntgenprojektionen (P1, ..., Pj) und/oder der tomographischen Schnitte (14) umfaßt und ein System (40) für den Zugriff auf diesen Rechner.

3. Verfahren nach Anspruch 1 oder 2, dadurch gekennzeichnet, daß ein erster Kollimator (15) am Ausgang der Quelle vorgesehen ist, um zur Durchführung von klassischer Tomographie die von der Quelle kommende Strahlung (5) zu kollimatieren.

4. Verfahren nach einem der Ansprüche 1 bis 3, dadurch gekennzeichnet, daß ein zweiter Kollimator (18) dem Detektor gegenüber vorgesehen ist, um als Maske für die direkt von der Quelle kommende Strahlung zu dienen.

5. Verfahren nach einem der Ansprüche 1 bis 4, dadurch gekennzeichnet, daß Archivierungseinrichtungen (38) der Röntgenprojektionen und/oder der tomographischen Schnitte des Objekts vorgesehen sind.

6. Verfahren nach einem der Ansprüche 1 bis 5, dadurch gekennzeichnet, daß das Ausles- bzw. Abfragsystem (30) wenigstens zwei Speicher umfaßt, einen Speicher (29), in dem Ausles- bzw. Abfragparameter des genannten Teils der Daten aufgezeichnet werden, und einen Speicher (31), in dem die ausgewählten Zeilen oder Spalten summiert bzw. kumuliert werden.

7. Verfahren zur zerstörungsfreien Prüfung eines Objekts, darin bestehend:
a) - das Objekt (4) unter verschiedenen Anstell- bzw. Einfallswinkeln einer ionsierenden Strahlung (5) auszusetzen, wobei das Objekt wenigstens eine relative Drehbewegung in bezug auf die Quelle-Detektor-Anordnung ausführt und jeder Anstell- bzw. Einfallswinkel einer Projektion entspricht,
b) - die von dem Objekt durchgelassene Strahlung mit Hilfe eines zweidimensionalen Detektors (6) mit mehreren Zeilen und Spalten zu detektieren, der in jedem Punkt analoge Signale liefert, die proportional zu der von dem Objekt durchgelassenen Strahlungsmenge sind.
c) - die genannten analogen Signale in digitale Daten umzuwandeln,
d) - diese digitalen Daten in einem ersten Speicher (24) abzuspeichern,
e) - diese digitalen Daten zu verarbeiten (36), um Röntgenprojektionen (P1, ..., Pj) des Objekts zu erstellen,
f) - wenigstens eine zu prüfende spezielle Zone des Objekts auszuwählen,
g) - für jede Projektion aus dem ersten Speicher die der genannten Zone entsprechenden digitalen Daten auszulesen (30) und sie zu summieren bzw. zu kumulieren,
h) - das der genannten Zone entsprechende Sinogramm der verschiedenen Projektionen aufgrund der ausgelesenen und summierten bzw. kumulierten Daten zu erstellen (32),
i) - diese Sinogramme zu verarbeiten (36), um den tomographischen Schnitt des Objekts (14) in der ausgewählten Zone zu rekonstruieren.

8. Verfahren nach Anspruch 7, dadurch gekennzeichnet, daß man aus dem ersten Speicher n' Zeilen oder Spalten von jeder Röntgenprojektion beiderseits der Zeile oder Spalte dieser Projektionen ausliest, die der Mitte der genannten speziellen Zone entspricht, und daß man sie bei jeder Projektion Spalte für Spalte oder Zeile für Zeile summiert bzw. kumuliert.

9. Verfahren nach Anspruch 7 oder 8, dadurch gekennzeichnet, daß man auf einem Bildschirm (26) die Röntgenprojektionen (P1, ..., Pj) und/oder die tomographischen Schnitte (14) sichtbar macht.

## Claims

1. Non-destructive testing apparatus with simultaneous acquisition of radiographic projection data for an object and tomographic section data for said object, comprising an ionizing radiation source-detector assembly (2-6), an object support (16) placed between the source and the detector, means (17) for ensuring at least one relative rotary movement of the support relative to the source-detector assembly, the detector being a bidimensional detector with several rows and several columns, supplying for each point of each radiographic projection (P1, ..., Pj) an analog signal proportional to the radiation quantity transmitted by the object (4), an analog-digital converter (22) for converting the signal supplied by the detector into digital data, a first memory (28) for storing said digital data, a system (30) for the extraction and accumulation of part of said stored data corresponding to at least one particular zone of the object to be tested, a second memory (32) for storing sinograms obtained from the data selected and accumulated by the extraction system and a processing system (36) for said sinograms for the construction of tomographic sections (14).

2. Apparatus according to claim 1, characterized in that the processing system incorporates a computer (36) for the control of the apparatus and the reconstruction of tomographic sections, controlling a display apparatus (26) for the radiographic projections (P1, ..., Pj) and/or tomographic sections (14) and an access system (40) to said computer.

3. Apparatus according to claim 1 or 2, characterized in that a first collimator (15) is provided at the outlet from the source for collimating the radiation (5) from said source for carrying out conventional tomography.

4. Apparatus according to any one of the claims 1 to 3, characterized in that a second collimator (18) is provided facing the detector in order to serve as a mask for the radiation directly coming from the source.

5. Apparatus according to any one of the claims 1 to 4, characterized in that there are filing means (38) for the radiographic projections and/or tomographic sections of the object.

6. Apparatus according to any one of the claims 1 to 5, characterized in that the extraction system (30) incorporates at least two memories, a memory (29) in which are recorded extraction parameters of said part of the data and a memory (31) in which are accumulated the selected rows or columns.

7. Process for the non-destructive testing of an object comprising:
a) subjecting an object (4) to ionizing radiation (5) under different incidences, the object having at least one relative rotary movement with respect to the source-detector assembly, each incidence corresponding to a projection,
b) detecting the radiation transmitted by the object with the aid of a bidimensional detector (6) having several rows and columns, supplying at each point analog signals proportional to the radiation quantity transmitted by the object,
c) converting (22) said analog signals into digital data,
d) storing said digital data in a first memory (24),
e) processing (36) the digital data in order to produce radiographic projections (P1, ..., Pj) of the object,
f) selecting at least one particular zone of the object to be tested,
g) extracting (30) from the first memory the digital data corresponding to said zone and accumulating the same for each projection,
h) producing (32) the sinogram corresponding to said zone from data extracted and accumulated from the different projections and
i) processing (36) said sinogram in order to reconstruct the tomographic section of the object (14) in the selected zone.

8. Process according to claim 7, characterized in that extraction takes place from the first memory of n' rows or columns of each radiographic projection on either side of the row or column of these projections corresponding to the centre of said particular zone and in that they are accumulated column by column or row by row in each projection.

9. Process according to claim 7 or 8, characterized in that on a display (26) are displayed the radiographic projections (P1, ..., Pj) and/or the tomographic sections (14).
